Europäisches Patentamt

European Patent Office

Office européen des brevets

(19)

(11) Publication number: **0 190 543**
**A1**

## (12) EUROPEAN PATENT APPLICATION

(21) Application number: **85850018.4**

(22) Date of filing: **17.01.85**

(51) Int. Cl.⁴: **A 61 F 5/04**

(30) Priority: **04.01.85 SE 8500034**

(43) Date of publication of application:
**13.08.85 Bulletin 86/33**

(84) Designated Contracting States:
**AT BE CH DE FR GB IT LI LU NL SE**

(71) Applicant: Hansson, Bertil
Dalgatan 16 D
S-591 60 Motala(SE)

(72) Inventor: Hansson, Bertil
Dalgatan 16 D
S-591 60 Motala(SE)

(74) Representative: Onn, Thorsten et al,
AB STOCKHOLMS PATENTBYRA Box 3129
S-103 62 Stockholm(SE)

(54) Emergency splint for different active-service purposes.

(57) A device for providing a support, especially at acute treatment of ruptures on upper arms, elbows, fore-arms, thigh-bones, knee-joints, lower legs and wrists injuries under active-service conditions. The support comprises an elongate underlaying layer of fabric (1), in the longitudinal direction of which laths (2) are arranged thereon so that a great stiffness is obtained in this direction. The laths are free from each other except for the connection to said layer so that bending is possible in a plane perpendicular to the longitudinal direction of the laths, the laths each being divided longitudinally into sections by means of recesses at agreeing places in order to enable folding of the support when not used. One of the recesses is provided with ring markings (5) at some distance from the edge of the device serving as a stop at slitting of the recess space for angular splinting of e.g. an elbow.

FIG.1

EP 0 190 543 A1

EMERGENCY SPLINT FOR DIFFERENT ACTIVE-SERVICE PURPOSES

This invention relates to a device for use with providing an emergency splint in connection with active-service treatment of fractures of upper arms, forearms, thigh-bones, knees, lower legs, elbows and wrist injuries. These active-service conditions can be of a military as well as civil nature and the essential function of the emergency splint is to hold the bone portions in such a correct position as possible with minimized reciprocal movement so that no further injuries result, especially in connection with a following transport to a hospital or the like.

For this purpose there are today so-called medical splints dating back to World War II. However, these are extremely awkward and must therefore be brought along separately, as they cannot be accomodated in a usual medical treatment kit bag. As is easily realized from this they will therefore sometimes be lacking on the spot and will not be used as required by circumstances at medical treatment in the field, e.g. in the Armed Forces, the Red Cross, as well as emergency medical treatment at motor races, mountain hikes etc. The fact is that these known medical splints are not used at all in the civil sector and they are not even found in the market despite the great use that one might have of these. The medical splints existing today comprise the so-called angular splint which is about 50 cm in length and intended for lower legs, forearms, elbows and upper arms. Moreover, there is an extension splint which is also about 50 cm in lenght and, above all, is used för knees and thigh-bones, sometimes called knee splint. Furthermore there is the wrist splint which is about 49 cm in length and intended for wrist and possibly forearm.

At the most usual type of fracture which is a fracture on the lower part of the leg the angular splint as well as the extension splint is preferably used to prevent bending of the knee, which is considered to be what is best. Besides the fact that these splints are clumsy and that several different types must be available they stand, moreover, moisture badly .

In order to give an idea of the present demand for improved devices for emergency splints it can be mentioned that at least 600 persons are injured in Sweden each working day. In traffic and in accidents in connection with leisure time activities 10-15 000 persons are injured annually and medical experts completely agree that the percentage of permanent injuries might be reduced considerably if it was possible to apply good emergency splints already at the scene of the accident. A certain part of the remaining injuries is due to movements during the transport to the hospital. Of course this is especially the case with active-service transports.

As a summary it can be said that there is today a great need of an emergency splint device that can be used with all types of ruptures, sits steadily and stably and protects against impacts. It must be possible to fix it by means of any available material, e.g. a triangular bandage, a waist belt, a string, a scarf, a shirt or the like. Moreover it must be so simple to apply the device that substantially anyone can do it and, moreover, the device must not be bulky but one must be able to pack it together with other things in a medical treatment kit bag or at another place. Furthermore, it must stand rain, snow, cold as well as heat. It is the object of the invention to meet the above-mentioned need, all requirements mentioned above being fulfilled. This is carried out according to the invention in that the device includes a flat, possibly elongate underlayer, in the longitudinal direction of which lath members are placed beside each other. In this way an emergency splint can be easily obtained which is flexible in one direction to be arranged around a leg, arm etc, whereas it will be very stiff in the longitudinal direction thanks to the laths forming together a more or less tubular structure. The laths need not be placed further away from each other than that the necessary flexibility is obtained. Of course the fabric need not be too soft, as a certain shearing between the laths and unsteadiness resulting from this might be possible. Besides, the laths need not be continuous, but are preferably provided with recesses at two positions in the longitudinal direction so that the emergency splint device can be folded up three-fold. It is then suitable that one of these recesses is wider so that the bandage can be folded with the laths against each other while the other recess need only consist of one slit, as the bandage can be turned in the opposite direction there. In this way the possibility is also obtained to provide a perpendicular support by slitting open the bandage from the sides at the wider one of the recesses. As the fabric is relatively stiff no flexibility that may be a disadvantage will appear between the different lath portions. The device of the invention is preferably made of plastic. It can then either be extruded in one piece or be manufactured from separate laths or profiles welded or glued to a supporting fabric.

In addition to the above-mentioned requirements being fulfilled the inventive object will be cheap to manufacture, and therefore it will be economically possible to use the bandage in all the connections where it may be needed.

Further advantages and properties of the invention will appear more closely from the description below in connection with the enclosed drawing. Fig. 1 is a top view of the supporting device according to the invention whereas, Fig.2 is a side view of the device and, finally, Fig. 3 is a cross section of the device.

The supporting device shown on the drawing is shown as lying flat on a support and has in this position a length of 611 mm and a width of 270 mm. Further below the supporting device consists of a fabric 1, on which laths 2 have been placed. These laths are placed consecutively three and three on a line with an interpace of 1 mm in longitudinal direction in one joint and with an interpace of 10 mm in the other. Laterally the laths 2 are placed at a distance of 1,25 mm from each other. In the middle of the emergency splint 31 mm are without laths.

When storing the supporting device it is first folded in the form of an S longitudinally until it becomes flat, and then it can be folded as an U around the space without laths between the groups of laths.

This embodiment of the invention can be manufactured in several ways, but one is pressure die casting the supporting device in one or more parts or extruding it.

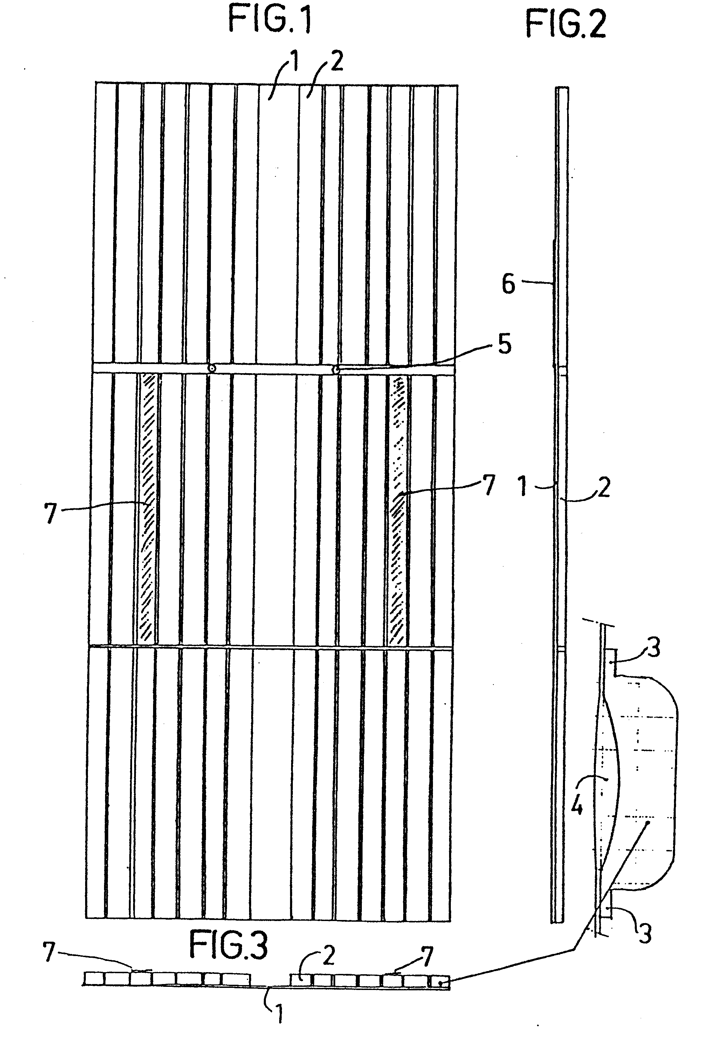

As is apparent from Fig. 1 hobbings or welds 5 are arranged in the supporting fabric at the broader interspace between the laths (longitudinally), in order to enable the arrangement of an angular support for splinting an elbow or foot. In such a use a slit is cut or made from the edge into the annular hobbing or weld 5 serving as a stop of the slit. Bur fasteners 5 and 7 on two laths in the entermediate section and on the underside, respectively, are then used to fix the angle. The number of injuries where this type of emergency splints has to be arranged is relatively small, and therefore the splints can be re-used in most cases. The normal case will be that the emergency splint is used in the whole of its length or, as an alternative, only 2/3 of its lengts, i.e. with one end placed doubly over the intermediate portion.

In order to keep the splint together in storage an elastic string or, as an alternative, a band intended for keeping the splint on its place in use can be employed.

The laths are attached to the fabric by means of lateral flanges 3, which have been high-frequency welded to the fabric. The fabric will then arch somewhat from the bottom of the lath between the flanges of the relative lath. In this way a padding 4 will be obtained which will make the splint more kind to the portion of the body around which it is to be placed, without disturbing the stiffness. If desired, special measures can be taken in manufacture to ensure a sufficient size of these cushions, e.g. by sucking down the fabric into the tool at welding by means of vacuum.

As fabric reinforced PVC-fabric has been used in the illustrative example, and the laths are here of microporous PVC. The laths can either be welded all around or only at their longitudinal edges.

The described embodiment is extremely simple to manufacture and the manufacture can also take place with a relatively simple apparatus already at small series. At larger series other methods may be found to be more economical, e.g. extusion in one single material (i.e. both fabric and laths).

WHAT I CLAIM IS:

1. A device for providing a support, especially at acute treatment of fractures of upper arms, elbows, forearms, tigh-bones, knee-joints, lower legs and wrist injuries under active service conditions, characterized in that it comprises a elongate underlaying layer of fabric or the like, in the longitudinal direction of which laths or profiles are arranged thereon so that a great stiffness is obtained in this direction, the laths being free from each other except for the connection to said layer so that bending is possible in a plane perpendicular to the longitudinal direction of the laths, the laths each being divided into sections longitudinally at corresponding places.

2. The device of claim 1, characterized in that the laths are divided into three sections.

3. The device of claim 1, characterized in that one of the recesses across the laths is so broad that the device can be folded with the laths facing each other.

4. The device of claim 2, characterized in that slits extending from the sides of the device are arranged between the lath portions at the broader recess.

5. The device of claim 3, characterized in that one of the recesses is provided at some distance from the edge of the device with punched holes or ring markings serving as a stop at slitting of the recess space for angular splinting of e.g. an elbow.

6. The device of any one of the preceding claims, characterized in that one or more laths are omitted at the longitudinal centre line of the device enabling double folding around this centre line of the device when not used, even if it is already folded in its other direction.

7. The device of any one of the preceding claims, characterized in that a padding or wadding is arranged at the rear side of each lath, e.g. in the form of an air-filled cushion, to provide a softer contact with injured portions.

8. The device of any one of the preceding claims, characterized in that it is made of plastic.

9. The device of claim 7, characterized in that the cushion is obtained at welding of the laths to the support by welding only around the edges of the laths.

10. The device of claims 4 or 5, characterized in that the adjacent sections in connection with the possible slit are provided with fixing means for reciprocal angular fixing (e.g. at elbow ruptures).

0190543

FIG.1

FIG.2

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| X | US-A-1 389 525 (J.K. MOSBY) <br> * Page 1, lines 80-92; figures 5,6 * | 1 | A 61 F 5/04 |
| X | US-A-2 785 672 (V.B. NAPOLI) <br> * Column 1, lines 66-72; column 2, lines 13-22; figure 4 * | 1,7,8 | |
| X | US-A-2 667 868 (L.A. SMYTH) <br> * Column 2, lines 1-5, 10-18; column 2, line 54 - column 3, line 13; figures 8,9 * | 1 | |
| A | US-A-3 232 289 (Ch.E. ZIMMERMAN) <br> * Column 2, lines 24-29, 38-47; column 3, lines 42-62; figures 1-3 * | 1 | TECHNICAL FIELDS SEARCHED (Int. Cl.4) |
| A | GB-A- 644 273 (C.V. FROST) <br> * Page 2, lines 17-28; claim * | 10 | A 61 F |
| A | DE-C- 811 256 (A. FERSTL) <br> * Page 2, lines 77-89; claims 1-3 * | 1 | |

The present search report has been drawn up for all claims

| Place of search | Date of completion of the search | Examiner |
|---|---|---|
| THE HAGUE | 04-04-1986 | SEDY, R. |